# DEMANDE DE BREVET EUROPEEN

(11) **EP 3 001 198 A1**
(43) Date de publication de la demande: **30.03.2016**
(21) Numéro de dépôt: 15182965.2
(22) Date de dépôt: 15.09.2003
(51) Int. Cl.: G01N 33/68, C12Q 1/02, G01N 33/80, G01N 33/53, A61K 39/395

(54) **TRAITEMENT DES PATHOLOGIES ECHAPPANT A LA REPONSE IMMUNE PAR DES ANTICORPS OPTIMISES**

(30) Priorité: 13.09.2002 FR 0211415; 13.09.2002 FR 0211416; 12.06.2003 FR 0307066
(62) Demande divisionnaire de: 03775438.9
(71) Demandeur: Laboratoire Français du Fractionnement et des Biotechnologies, 91940 Les Ulis (FR)
(72) Inventeur: DE ROMEUF, Christophe, 59000 Lille (FR); GAUCHER, Christine, 59320 Sequedin (FR); JORIEUX, Sylvie, 59650 Villeneuve d'Ascq (FR); BOUREL, Dominique, 59110 La Madeleine (FR)
(74) Mandataire: Regimbeau

(57) **Abrégé**

La présente invention concerne l'utilisation d'anticorps monoclonaux chimériques, humanisés ou humains optimisés qui sont produits dans des lignées cellulaires sélectionnées, lesdits anticorps présentant une forte affinité pour le récepteur CD16 des cellules effectrices du système immunitaire mais également la propriété d'induire la sécrétion de cytokines et d'interleukines, en particulier l' IFNγ ou l'IL2 pour le traitement de pathologies pour lesquelles les cellules cibles n'expriment qu'une faible densité antigénique et dans lesquelles les cellules effectrices ne peuvent être recrutées qu'en faible quantité.

## Description

La présente invention concerne l'utilisation d'anticorps monoclonaux chimériques, humanisés ou humains optimisés qui sont produits dans des lignées cellulaires sélectionnées, lesdits anticorps présentant une forte affinité pour le récepteur CD16 des cellules effectrices du système immunitaire mais également la propriété d'induire la sécrétion de cytokines et d'interleukines, en particulier l'IFNγ ou l'IL2, pour le traitement de pathologies pour lesquelles les cellules cibles n'expriment qu'une faible densité antigénique et dans lesquelles les cellules effectrices ne peuvent être recrutées qu'en faible quantité.

L'immunothérapie au moyen d'anticorps monoclonaux est en passe de devenir un des aspects les plus importants de la médecine. En revanche, les résultats obtenus lors d'essais cliniques apparaissent contrastés. En effet, il peut s'avérer que l'anticorps monoclonal ne soit pas suffisamment efficace. De nombreux essais cliniques sont arrêtés pour diverses causes telles que le manque d'efficacité et des effets secondaires incompatibles avec une utilisation en thérapie clinique. Ces deux aspects sont étroitement liés sachant que des anticorps peu actifs sont administrés à forte dose pour compenser et obtenir une réponse thérapeutique. L'administration de fortes doses induit non seulement des effets secondaires mais est économiquement peu viable.

Ces problèmes sont majeurs dans l'industrie des anticorps monoclonaux chimériques humanisés ou humains.

Or, ce problème est exacerbé pour un certain nombre de pathologies pour lesquelles la densité antigénique exprimée par les cellules cibles est faible et/ou le faible nombre de cellules effectrices disponibles et activées est limité, rendant ainsi techniquement impossible l'utilisation d'anticorps à visée thérapeutique avec les anticorps actuellement disponibles. Par exemple, dans le Syndrome de Sézary, l'antigène spécifique, KIR3DL2, est faiblement exprimé (seulement environ 10 000 molécules). L'expression des antigènes tumoraux peut également être régulée négativement, comme HER2-neu dans le cancer du sein. Par ailleurs, lorsque l'on cherche à inhiber l'angiogénèse via le ciblage du VEGFR2, peu de cibles moléculaires sont effectivement accessibles car le récepteur est internalisé. De même, les peptides spécifiques d'antigènes de tumeurs présentés par les molécules HLA de classe 1 ou classe 2, par exemple dans le cas de carcinomes, mélanomes, cancers ovarien, cancers de la prostate sont généralement peu exprimés à la surface des cellules cibles tumorales. Enfin, une autre situation peut se trouver lors d'infections virales dans lesquelles les cellules infectées par certains virus (VHB, VHC, VIH) n'expriment que peu de molécules virales sur leur membrane.

Ce problème se pose aussi pour toutes les pathologies qui présentent une diminution du nombre de cellules NK, ou de leur activité ou de leur nombre de CD16 (Cavalcanti M et al, Irreversible cancer cell-induced functional anergy and apoptosis in resting and activated NK cells, Int J Oncol 1999 Feb;14(2):361-6). On peut citer à titre d'exemple, les leucémies myéloides chroniques (Parrado A. et al., Natural killer cytotoxicity and lymphocyte subpopulations in patients with acute leukaemia, Leuk Res 1994 Mar;18(3):191-7), les pathologies liées à l'environnement visant notamment les personnes exposées aux biphényles polychlorinés (Svensson BG. et al., Parameters of immunological competence in subjects with high consumption of fish contaminated with persistent organochlorine compounds, Int Arch Occup Environ Health 1994;65(6):351-8), les maladies infectieuses, notamment la tuberculose (Restrepo LM. et al, Natural killer cell activity in patients with pulmonary tuberculosis and in healthy controls, Tubercle 1990 Jun;71(2):95-102), le syndrome de la fatigue chronique (CFS) (Whiteside TL, Friberg D, Natural killer cells and natural killer cell activity in chronic fatigue syndrome, Am J Med 1998 Sep 28;105(3A):27S-34S), et toutes les infections parasitaires comme par exemple les schistosomules (Feldmeier H, et al, Relationship between intensity of infection and immunomodulation in human schistosomiasis. II. NK cell activity and in vitro lymphocyte proliferation, Clin Exp Immunol 1985 May; 60(2):234-40).

Ainsi, l'objectif est d'obtenir de nouveaux anticorps présentant une meilleure efficacité comparée aux anticorps actuels, ce qui permettrait d'envisager leur utilisation en thérapie pour les pathologies présentant peu de cibles moléculaires exprimées ou une faible densité antigénique ainsi qu'un nombre limité de cellules effectrices capables d'être activées.

Nous avions montré dans notre demande WO 01/77181 (LFB) l'importance de sélectionner des lignées cellulaires permettant de produire des anticorps présentant une forte activité ADCC via le FcγRIII (CD16). Nous avions trouvé que la modification de la glycosylation du fragment constant des anticorps produits dans des lignées de myélomes de rat telle que YB2/0 conduisait à améliorer l'activité ADCC. Les structures glycanniques desdits anticorps sont de type biantennées, avec des chaînes courtes, une faible sialylation, des mannoses et GlcNAc du point d'attache terminaux non intercalaires, et une faible fucosylation.

Or, dans le cadre de la présente invention, nous avons découvert que l'avantage de présenter une forte affinité pour le CD16 peut encore être renforcé par des conditions supplémentaires visant à produire des anticorps qui induisent également la production de cytokines, notamment la production d'IFNγ ou l'IL2 par les cellules du système immunitaire.

Les deux caractéristiques précitées se complémentent. En effet, la production d' IFNγ ou l'IL2 induite par les anticorps sélectionnés par le procédé de l'invention peut renforcer l'activité cytotoxique. Le mécanisme d'action d'une telle activation tient probablement à une régulation positive autocrine des cellules effectrices. On peut postuler que les anticorps se lient au CD16 provoquant une activité cytotoxique mais également induisent la production de l'IFNγ ou l'IL2 qui au final conduit à augmenter encore davantage l'activité cytotoxique.

Nous montrons ici que les anticorps optimisés de l'invention conservent une bonne efficacité même lorsque la densité antigénique est faible ou le nombre de cellules effectrices limité. Ainsi, à des doses compatibles avec une utilisation en thérapie clinique, il est désormais possible de traiter des pathologies pour lesquelles un traitement par anticorps n'était pas envisageable à ce jour.

### Description

Ainsi, l'invention concerne l'utilisation d'un anticorps monoclonal chimérique, humanisé ou humain optimisé caractérisé en ce que :
a) il est produit dans une lignée cellulaire sélectionnée pour ses propriétés de glycosylation du fragment Fc d'un anticorps, ou
b) la structure glycannique du Fcgamma a été modifiée ex vivo, et/ou
c) sa séquence primaire a été modifiée de façon à augmenter sa réactivité vis à vis des récepteurs Fc ;
   ledit anticorps présentant i) un taux ADCC via le FcγRIII (CD16) supérieur à 50 %, de préférence supérieur à 100 % pour un ratio E/T (cellules effectrices/cellules cibles) inférieur à 5/1, de préférence inférieur à 2/1, comparé au même anticorps produit dans une lignée CHO ; et ii) un taux de production d'au moins une cytokine par une cellule effectrice du système immunitaire exprimant le récepteur CD16 supérieur à 50 %, 100 % ou de préférence supérieur à 200 % comparé au même anticorps produit dans une lignée CHO;
   pour la préparation d'un médicament destiné au traitement de pathologies pour lesquelles le nombre de sites antigéniques ou la densité antigénique sont faibles, les antigènes sont peu accessibles aux anticorps, ou encore pour lesquelles le nombre de cellules effectrices activées ou recrutées est faible.

Avantageusement, le nombre de sites antigéniques est inférieur à 250 000, de préférence inférieur à 100 000 ou 50 000 par cellule cible.

Lesdites cytokines libérées par les anticorps optimisés sont choisies parmi des interleukines, des interférons et des facteurs de nécrose tissulaire (TNF).

Ainsi, l'anticorps est sélectionné pour sa capacité d'induire la sécrétion d'au moins une cytokine choisie parmi IL-1, IL-2, IL-3, IL-4, IL-5, IL-6, IL-7, IL-8, IL-9, IL-10,... TNFα, TGFβ, IP10 et IFNγ par les cellules effectrices du système immunitaire exprimant le récepteur CD 16.

De préférence, l'anticorps sélectionné présente la capacité d'induire la sécrétion d' IFNγ ou d'IL2 par les cellules effectrices du système immunitaire exprimant le récepteur CD16 ou de l'IL2 par la cellule Jurkat CD16 pour un faible nombre de sites antigéniques présents à la surface des cellules cibles ou pour un faible nombre d'antigènes accessibles aux anticorps. Le taux IFNγ ou d'IL2 sécrété reflète la qualité de l'anticorps fixé par le récepteur CD16 quant à son intégrité (fonction Fc) et à son efficacité (site antigénique) de liaison à l'antigène. En outre, la sécrétion d'IFNγ ou d'IL2 par les cellules du système immunitaire peut activer l'activité cytotoxique des cellules effectrices. Ainsi, les anticorps de l'invention sont également utiles pour le traitement de pathologies pour lesquelles le nombre de cellules effectrices activées ou recrutées est faible.

Les cellules effectrices peuvent exprimer un CD16 endogène ou être transformées. On entend par cellule transformée, une cellule modifiée génétiquement de sorte à exprimer un récepteur, en particulier le récepteur CD16.

Dans un mode de réalisation particulier, l'anticorps de l'invention est capable d'induire la sécrétion d'au moins une cytokine par une cellule leucocytaire, en particulier de la famille des NK (natural killer) ou par des cellules du groupe monocytes-macrophages. De préférence, on utilise pour la sélection des anticorps une lignée Jurkat transfectée avec un vecteur d'expression codant pour le récepteur CD16 comme cellule effectrice. Cette lignée est particulièrement avantageuse car elle est immortalisée et se développe indéfiniment dans des milieux cultures. Le taux d'interleukine IL2 sécrétée reflète la qualité de l'anticorps fixé par le récepteur CD16 quant à son intégrité (fonction Fc) et à son efficacité (site antigénique) de liaison à l'antigène.

Dans un autre mode de réalisation, l'anticorps optimisé peut être préparé après avoir été purifié et/ou modifié ex vivo par modification de la structure glycannique du fragment Fc. A cet effet, on peut utiliser tout moyen chimique, chromatographique ou enzymatique approprié pour modifier la structure glycannique des anticorps.

Dans un autre mode de réalisation, l'anticorps peut être produit par des cellules de lignées de myélomes de rat, en particulier YB2/0 et ses dérivées. D'autres lignées peuvent être sélectionnées pour leurs propriétés de produire les anticorps définis ci-dessus. On pourra tester par exemple les cellules lymphoblastoïdes humaines, les cellules d'insectes et les cellules de myélomes murines. La sélection peut également être appliquée à l'évaluation des anticorps produits par des plantes transgéniques ou de mammifères transgéniques. A cet effet, la production dans CHO sert de référence (CHO étant employée pour la production d'anticorps médicament) pour comparer et sélectionner les systèmes de production conduisant aux anticorps selon l'invention.

La structure glycannique générale de l'anticorps correspond à un type biantenné, avec des chaînes courtes, une faible sialylation, des mannoses et GlcNAc du point d'attache terminaux non intercalaires, et une faible fucosylation. Dans ces anticorps, le taux de GlcNac intermédiaire est non nul.

Ainsi, l'invention vise l'utilisation d'un anticorps décrit ci-dessus pour la préparation d'un médicament destiné au traitement d'une pathologie échappant à la réponse immune notamment choisie parmi la maladie hémolytique du nouveau né, le Syndrome de Sézary, les leucémies myéloides chroniques, les cancers dont les cibles antigéniques sont faiblement exprimées, notamment le cancer du sein, les pathologies liées à l'environnement visant notamment les personnes exposées aux biphényles polychlorinés, les maladies infectieuses, notamment la tuberculose, le syndrome de la fatigue chronique (CFS), les infections parasitaires comme par exemple les schistosomules.

### Légendes et titres des figures:

Figure 1: ADCC sur hématies : comparaison hématies normales (N) versus hématies sur-exprimant l'antigène Rhésus (GR6) (Teg 500 µg / puits, ADCC 375 03 017)
Figure 2: Activité ADCC induite par les anticorps chimériques anti-HLA-DR exprimés dans CHO ou YB2/0 en fonction du ratio E/T.
Figure 3: Influence du nombre d'antigènes HLA-DR exprimés sur Raji (blocage par Lym-1) sur l'activité ADCC induite par les anticorps chimériques anti-HLA-DR exprimés dans CHO(carré) ou YB2/0 (triangle).
Figure 4: Influence du nombre d'antigènes HLA-DR exprimés sur Raji (blocage par Lym-1) sur l'activation de Jurkat CD16 (IL2) induite par les anticorps chimériques anti-HLA-DR exprimés dans CHO(carré) ou YB2/0 (triangle).
Figure 5: Influence du nombre d'antigènes CD20 exprimés sur Raji (blocage par CAT 13) sur l'activation de Jurkat CD16.
Figure 6: Corrélation entre le test ADCC et la sécrétion d'IL2 par Jurkat CD16.
Figure 7: IL8 sécrétée par les CMN en présence ou absence de cible
Figure 8: Sécrétion de cytokines par les CMN induite par les anticorps anti-Rhésus (valeur sans cible déduite) Tox 324 03 062
Figure 9: Sécrétion de cytokines par les polynucléaires induite par les anticorps anti Rhésus.
Figure 10: Sécrétion de cytokines par les NK induite par les anticorps anti-Rhésus
Figure 11: Sécrétion de TNF alpha par les cellules NK, induite par les anticorps anti-CD20 et anti-HLA-DR exprimés dans CHO et YB2/0 (324 03 082).
Figure 12: Sécrétion d'IFN gamma par les cellules NK, induite par les anticorps anti-CD20 et anti-HLA-DR exprimés dans CHO et YB2/0 (324 03 082).

### Exemple 1 : ADCC induit par des anticorps anti-Rhésus en fonction du nombre de sites antigéniques.

La même séquence codant pour une IgG1 spécifique de l'antigène Rhésus D est transfectée dans CHO et YB2/0. L'activité cytotoxique des anticorps est comparée vis à vis des hématies Rhésus positif exprimant à leur surface differentes quantités d'antigène Rhésus, c'est à dire : hématies O+ normales (10-20 000 sites) et hématies sur-exprimant l'antigène rhésus (>60000 sites).

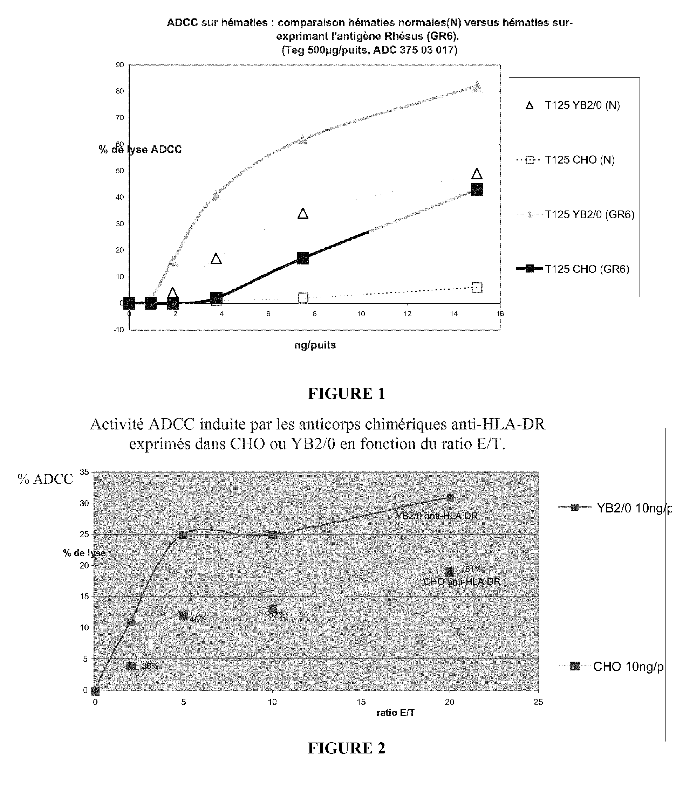

Les résultats sont présentés à la Figure 1 :
L'activité ADCC des anticorps exprimés dans CHO (triangle) ou YB2/0 (carré) sur des hématies normales (N, vide) ou sur-exprimant l'antigène Rhésus (GR6, plein) sont comparées.

La différence d'activité ADCC entre l'anticorps exprimé dans CHO et l'anticorps exprimé dans YB2/0 est moindre sur les hématies sur-exprimant l'antigène Rhésus surtout aux fortes quantités d'anticorps et augmente au fur et à mesure que le nombre de site antigénique décroît. Ainsi, plus la densité antigénique baisse, plus la différence d'activité ADCC entre l'anticorps produit dans YB2/0 et l'anticorps produit dans CHO augmente.

### Exemple 2 : ADCC induit par des anticorps anti-HLA DR en fonction de la quantité d'effecteurs

La même séquence codant pour une IgG1 spécifique de l'antigène HLA-DR est transfectée dans CHO et YB2/0. L'activité cytotoxique des anticorps est comparée vis à vis de la cellule Raji en présence de différents ratios effecteurs/cibles (voir Figure 2).

La différence d'activité cytotoxique entre l'anticorps optimisé exprimé par YB2/0 et CHO s'accroît au fur et à mesure que le ratio E/T diminue. Ainsi, pour les ratios suivants, 20/1 ; 10/1 ; 5/1 ; et 2/1, le pourcentage relatif de lyse induit par l'anticorps exprimé dans CHO (100% étant la valeur de l'anticorps exprimé dans YB2/0 pour chaque ratio) est de 61%, 52%, 48% et 36% respectivement.

L'anticorps exprimé dans YB2/0 s'avère plus cytotoxique que lorsqu'il est produit par CHO dans des conditions de faibles quantités d'effecteurs.

### Exemple 3 : ADCC induit par des anticorps anti-HLA DR en fonction de la quantité d'antigènes accessibles

La même séquence codant pour une IgG1 spécifique de l'antigène HLA-DR est transfectée dans CHO et YB2/0. L'activité cytotoxique des anticorps est comparée vis à vis de la cellule Raji en présence de différents ratios effecteurs/cibles (ratio E/T).

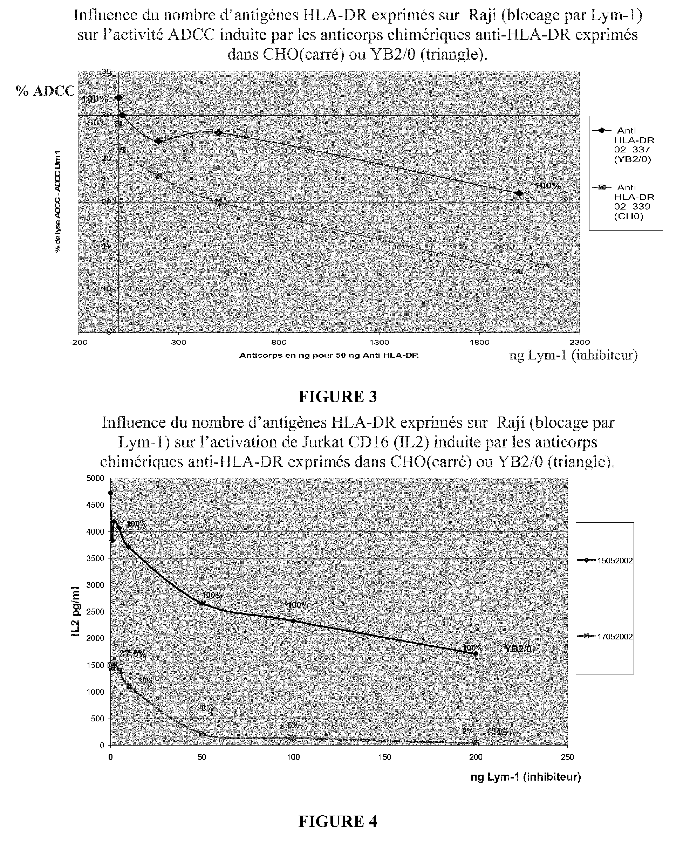

L'activité cytotoxique des anticorps est comparée vis à vis de cellules Raji dont les sites antigéniques ont été préalablement bloqués avec des quantités croissantes d'un anticorps murin inactif (non cytotoxique) anti-HLA-DR, de façon à avoir un nombre décroissant d'antigènes HLA-DR disponibles vis à vis des anticorps à évaluer (voir Figure 3).

Moins il y a de sites antigéniques disponibles, plus la différence d'activité cytotoxique entre l'anticorps optimisé produit dans YB2/0 et l'anticorps produit dans CHO augmente. Cela indique qu'une des applications de l'anticorps optimisé peut concerner des cellules cibles exprimant à leur surface un antigène peu exprimé reconnu par l'anticorps thérapeutique. Ceci procure un net avantage thérapeutique vis à vis d'un anticorps exprimé dans une cellule de type CHO.

### Exemple 4 : Production d'IL2 par Jurkat CD16 induite par des anticorps anti-HLA DR en fonction de la quantité d'antigènes accessibles

La même séquence codant pour une IgG1 spécifique de l'antigène HLA-DR est transfectée dans CHO et YB2/0. L'activation de la cellule effectrice (sécrétion d'IL2 par Jurkat CD16) induite par les anticorps est comparée vis à vis de cellules Raji dont les sites antigéniques ont été préalablement bloqués avec des quantités croissantes d'un anticorps murin anti-HLA-DR, de façon à avoir un nombre décroissant d'antigène HLA-DR disponible vis à vis des anticorps à évaluer (voir Figure 4).

Ces résultats montrent également que moins il y a de sites antigéniques disponibles, plus la différence d'activation des cellules effectrices entre l'anticorps optimisé produit par YB2/0 et l'anticorps produit dans CHO augmente.

### Exemple 5 : ADCC induit par des anticorps anti-CD20 en fonction de la quantité d'antigènes.

Les résultats obtenus avec l'anti-CD20 en ADCC confirment ceux obtenus avec les anti-HLADR, c'est à dire que moins il y a de sites antigéniques disponibles et exprimés à la surface des cellules cibles, plus la différence d'activation des cellules effectrices entre l'anticorps optimisé produit par YB2/0 et l'anticorps produit dans CHO augmente.

### Exemple 6 : Production d'IL2 par Jurkat CD16 induite par des anticorps anti-CD20 en fonction de la quantité d'antigènes accessibles

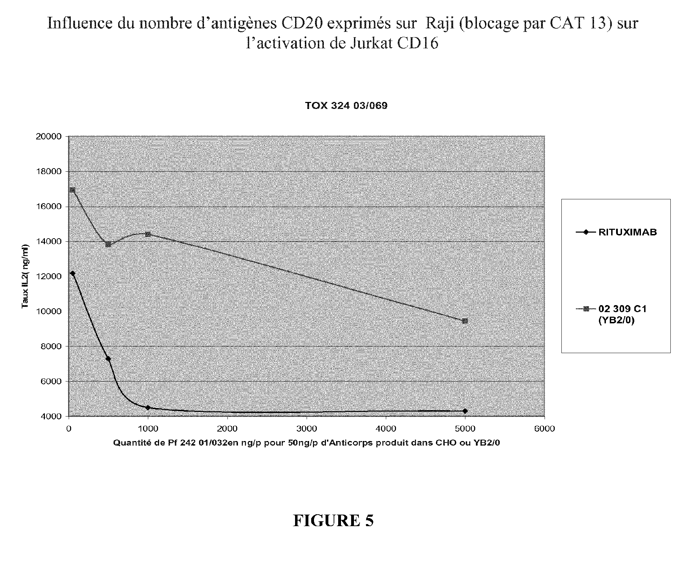

La même séquence codant pour une IgG1 spécifique de l'antigène CD20 est transfectée dans CHO et YB2/0. L'activation de la cellule effectrice (sécrétion d'IL2 par Jurkat CD16) induite par les anticorps est comparée vis à vis de cellules Raji dont les sites antigéniques ont été préalablement bloqués avec des quantités croissantes d'un anticorps murin inactif anti-CD20, de façon à avoir un nombre décroissant d'antigène CD20 disponibles vis à vis des anticorps à évaluer (voir Figure 5).

Moins il y a de sites antigéniques disponibles, plus la différence d'activation des cellules Jurkat CD16 induite par l'anticorps optimisé produit par YB2/0 et l'anticorps produit dans CHO augmente. Cela indique qu'une cellule exprimant une faible densité antigénique peut néanmoins induire l'activation d'une cellule effectrice via un anticorps optimisé. Cette capacité est beaucoup plus restreinte voire nulle avec un anticorps exprimé dans CHO.

Les applications thérapeutiques de l'anticorps optimisé c'est à dire produit dans YB2/0, peuvent ainsi concerner des cellules cibles exprimant à leur surface un antigène peu exprimé.

En conclusion, les anticorps optimisés s'avèrent particulièrement utiles pour des applications thérapeutiques lorsque les cellules cibles expriment à leur surface peu d'antigènes, et ceci quel que soit l'antigène.

### Exemple 7 : Corrélation in vitro entre ADCC et libération d'IL-2 par la cellule Jurkat CD16.

Pour cette étude, 3 anticorps monoclonaux anti-D ont été comparés.

L'anticorps monoclonal (Mab) DF5-EBV a été produit par des Lymphocytes B humain obtenus chez un donneur immunisé D-négatif et immortalisés par transformation avec l'EBV. Cet anticorps a été utilisé comme contrôle négatif étant donné que lors d'un essai clinique, il s'est montré incapable d'éliminer les globules rouges Rhésus positif de la circulation.

L'anticorps monoclonal (Mab) DF5-YB2/0 a été obtenu en exprimant la séquence primaire de DF5-EBV dans la lignée YB2/0. L'anticorps monoclonal R297 et d'autres anticorps recombinants ont également été exprimés dans YB2/0.

Les anticorps sont testés in vitro pour leur capacité à induire une lyse des globules rouges traités à la papaïne en utilisant des cellules mononucléées (PBL) comme effecteur.

Tous les tests ont été effectués en présence d'immunoglobulines humaines (IVIg) de sorte à reconstituer les conditions physiologiques.

On pense que les IVIg se lient avec une haute affinité au FcgammaRI (CD64). Les deux Mab DF5-YB2/0 et R297 induisent une lyse des globules rouges à un niveau comparable à celui des anticorps polyclonaux WinRho. En revanche, le Mab DF5-EBV est complètement inefficace.

Dans une deuxième série d'expérience, des cellules NK purifiées et des globules rouges non traités ont été utilisés comme effecteurs et cibles respectivement. Après 5 heures d'incubation, les Mabs antiD-R297 et DF5-YB2/0 se sont montrés capables de provoquer la lyse des globules rouges, alors que DF5-EBV reste inefficace.

Dans ces deux expériences, la lyse des globules rouges a été inhibée par le Mab 3G8 dirigé contre le FcgammaRIII (CD16).

En résumé, ces résultats démontrent que l'ADCC provoquée par le Mab R297 et le Mab DF5-YB2/0 implique le FcgammaRIII exprimé à la surface des cellules NK.

Dans le cadre de l'invention, une troisième série d'expériences a été réalisée en utilisant un test in vitro à l'aide des cellules Jurkat CD16 pour évaluer l'efficacité d'anticorps anti-D. Les Mab ont été incubés pendant la nuit avec des globules rouges Rhésus positif et des cellules Jurkat CD16. La libération d'IL-2 dans les surnageants a été évaluée par ELISA.

Une forte corrélation entre l'ADCC et l'activation des cellules Jurkat (production d'Il2) a été observée, ce qui implique que ce test peut être utilisé pour faire la discrimination des Mabs anti-D en fonction de leur réactivité envers FcgammaRIII ( CD16).

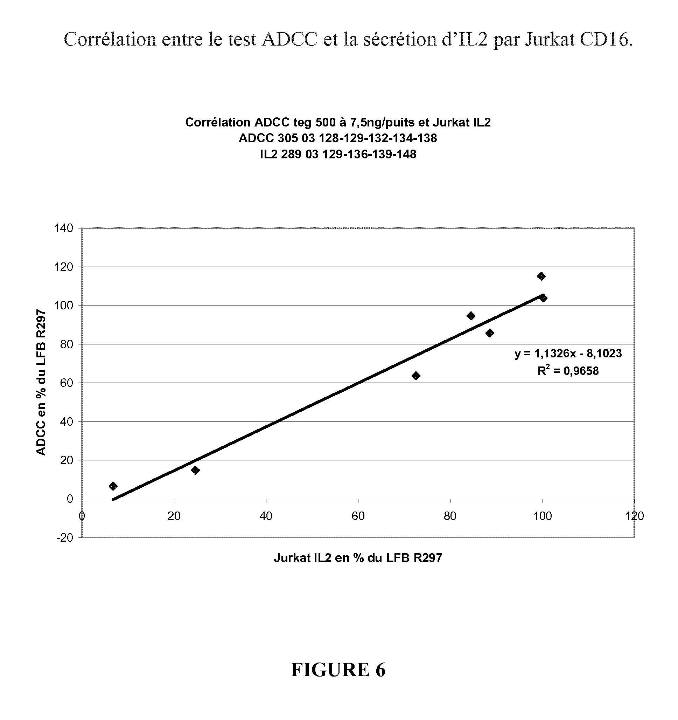

Les mêmes échantillons sont évalués en ADCC et dans le test Jurkat IL2. Les résultats sont exprimés en pourcentage par rapport à l'anticorps de référence "anti-D R297". La courbe de corrélation entre les 2 techniques a un coefficient r2=0.9658 (figure 6).

En conclusion, ces données montrent l'importance des modifications post-traductionnelles de la structure des anticorps et leur impact sur l'activité ADCC spécifique du FcgammaRIII (CD16). La libération de cytokines telles que IL-2 par les cellules Jurkat CD16 reflète cette activité.

### Exemple 8 : activation de cellules NK et production d'IL2 et d'IFNγ

Modèle de mise au point : lignée cellulaire Jurkat transfectée avec le gène codant pour le récepteur CD16. Applications : renforcement d'une réponse anti-tumorale. L'IL2, produite par les cellules effectrices activées par des immuns complexes antigène-anticorps, induit une activation des lymphocytes T et des cellules NK pouvant aller jusqu'à une stimulation de la prolifération cellulaire. L'IFNγ stimule l'activité des CTLs et peut renforcer l'activité des macrophages.

### Exemple 9 : activation de monocytes macrophages et production de TNF et d'IL-1Ra

Applications : renforcement de la phagocytose et induction de propriétés anti-inflammatoires. Le TNF, produit par les cellules effectrices activées par des immuns complexes antigène-anticorps, stimule la prolifération des macrophages et des lymphocytes infiltrant les tumeurs. L'IL-1Ra est une cytokine qui entre en compétition avec l'IL1 au niveau de son récepteur et exerce ainsi un effet anti-inflammatoire.

### Exemple 10 : activation de cellules dendritiques et production d'IL10

Applications : induction d'une tolérance spécifique à certains antigènes. L'IL10 est une molécule inhibitrice de l'activation de différentes cellules effectrices et de la production de cytokines. Ainsi l'IL10 produite par les cellules effectrices activées par des immuns complexes antigène-anticorps peut avoir un rôle régulateur de l'activité cytotoxique des anticorps vis à vis de cellules normales, mais exprimant des antigènes communs avec les cellules cibles visées, et également moduler les effets du TNF alpha.

### Exemple 11 : Induction de la sécrétion de cytokines par différentes cellules effectrices.

Trois populations cellulaires ont été étudiées : **les polynucléaires, les cellules mononuclées et les cellules NK.** L'induction de la synthèse de cytokines par des anticorps est dépendante de la présence de la cible. Il y a peu de différences dans la capacité de l'anticorps anti-D R297 et de l'anticorps polyclonal à induire la production de différentes cytokines. Par contre, AD1 est très souvent non inducteur de sécrétion de cytokines.

### Résultats :

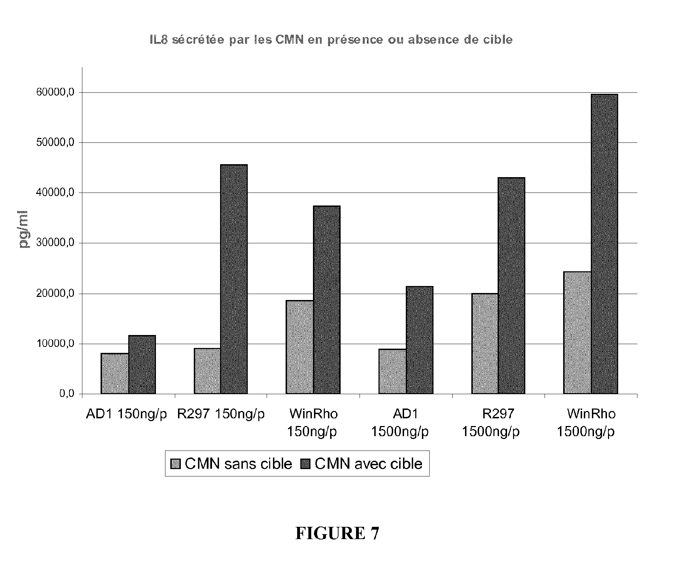

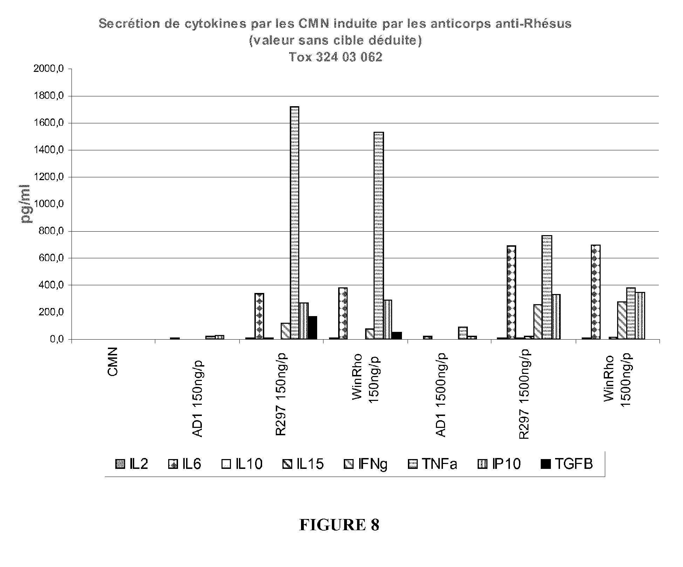

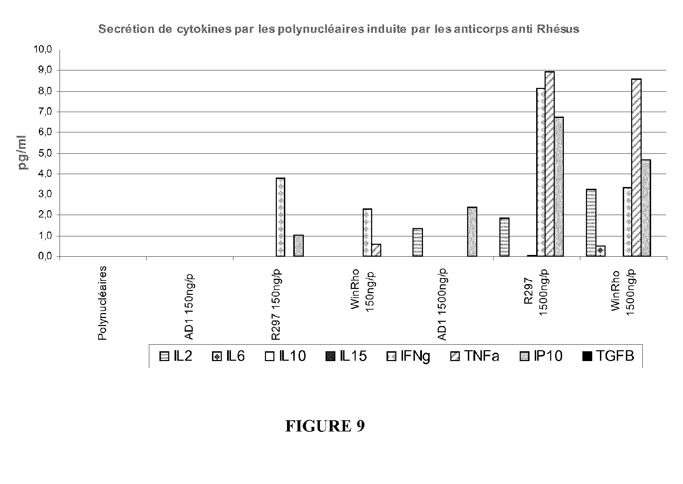

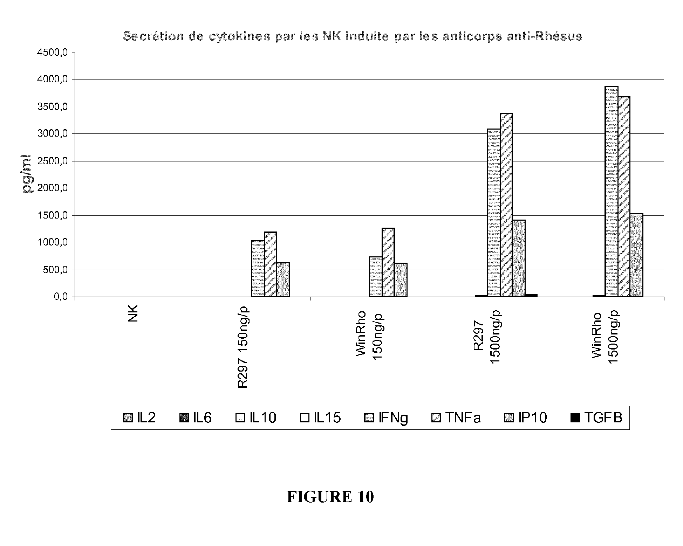

11.1 L'anticorps monoclonal R297 et l'anticorps polyclonal WinRho induisent une sécrétion importante d'IL8 en présence de cellules mononucléées. Cette sécrétion est dépendante de la concentration d'anticorps et de la présence de la cible antigénique, c'est à dire des hématies Rh positif. L'anticorps AD1 est beaucoup moins apte à induire la production d'IL8 (figure 7).
   En présence de cellules mononucléées et d'hématies Rhésus positif, l'anticorps monoclonal R297 et l'anticorps polyclonal anti-D WinRho induisent une sécrétion importante de TNF alpha, moins forte bien que supérieure à celles induites par AD1 d'IL6, d'IFN gamma, d'IP10, de TNF alpha et TGF Beta. En présence de plus forte concentration d'anticorps, la sécrétion dIL6, d'IFN gamma, d'IP10 augmente, mais celle de TNF alpha et le TGF Beta décroît (figure 8).
11.2 L'anticorps monoclonal R297 et l'anticorps polyclonal anti-D WinRho induisent une sécrétion très faible, mais supérieure à AD1, d'IL2, d'IFN gamma, d'IP10 et de TNF par les polynucléaires. Cette sécrétion est dépendante de la concentration d'anticorps (figure 9).
11.3 L'anticorps monoclonal R297 et l'anticorps polyclonal anti-D WinRho induisent une sécrétion importante d'IFN gamma, d'IP10 et de TNF par les cellules NK. Cette sécrétion est dépendante de la concentration d'anticorps (figure 10).

### Exemple 11 : Anticorps anti-CD 20 et anti-HLA DR chimériques optimisés produits dans YB2/0

### Introduction

Nos premiers résultats ont montré que les anticorps anti-D produits dans YB2/0 ainsi que les anticorps polyclonaux utilisés en clinique induisaient la production de cytokines, en particulier de TNF alpha et d'interféron gamma (IFN gamma) à partir de cellules NK purifiées ou de cellules mononucléées. Par contre d'autres anticorps anti-D, produits dans d'autres lignées cellulaires sont négatifs en ADCC et se sont révélés incapables d'induire une sécrétion de cytokines.

Les résultats complémentaires ci dessous montrent que ce mécanisme n'est pas exclusif aux anti-D en présence d'hématies Rhésus positif mais s'applique également aux anticorps anti-CD20 et anti-HLA DR exprimés dans YB2/0. L'expression dans CHO confère à l'anticorps des propriétés activatrices moins importantes. Ceci est en corrélation avec les résultats obtenus en ADCC.

### Matériel

### Anticorps.

Anti-CD20 : l'anticorps chimérique anti-CD20 transfecté dans YB2/0 est comparé à un anticorps commercial anti-CD20 produit dans CHO (Rituxan).

Anti-HLA DR : la même séquence codant pour l'anticorps chimérique anti-HLA DR est transfectée dans CHO (B11) ou YB2/0 (4B7).

*Cellules cibles :* cellules Raji exprimant à leur surface l'antigène CD20 et HLA-DR *Cellule effectrices* : cellules NK humaines purifiées par sélection négative à partir de poche de sang humain.

### Méthode

Différentes concentrations d'anticorps anti-CD20 ou anti-HLA DR sont incubées avec les cellules Raji et les cellules NK. Après 16 heures d'incubation, les cellules sont centrifugées. Les surnageants sont dosés en TNF alpha et en IFN gamma.

### Résultats :

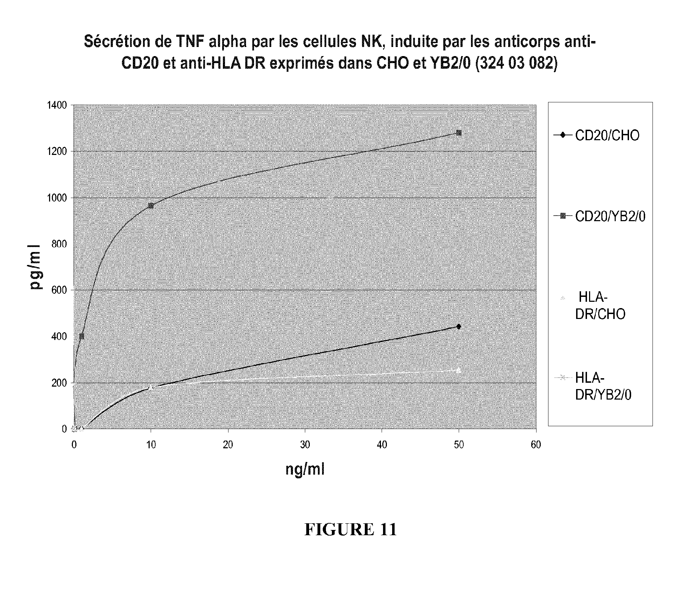

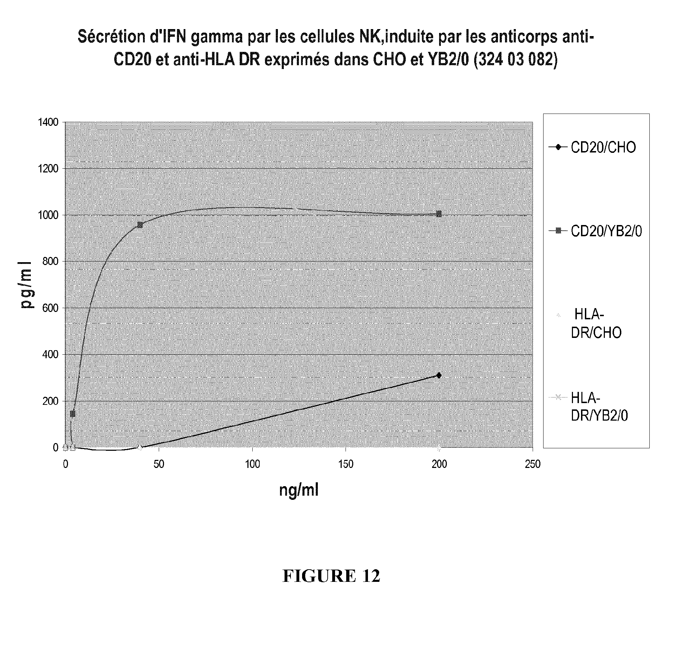

*1) TNF alpha :* Les résultats sont exprimés en pg/ml de TNF alpha dosé dans les surnageants. En abscisse figurent les différentes concentrations d'anticorps ajoutées dans le mélange réactionnel (figure 11).
   Les anticorps anti-CD 20 et anti-HLA DR chimériques produits dans YB2/0 induisent des taux plus importants de TNF en présence de leur cible (Raji) par rapport aux mêmes anticorps produits dans CHO. La quantité de TNF alpha est bien dose dépendante de la concentration d'anticorps ajouté. A 10ng/ml d'anticorps on induit 5 fois plus de TNF alpha avec les anticorps produits dans YB2/0 par rapport aux anticorps produits dans CHO.
*2) IFN gamma:* Les résultats sont exprimés en pg/ml d'IFN gamma dosé dans les surnageants. En abscisse figurent les différentes concentrations d'anticorps ajoutées dans le mélange réactionnel (figure 12).
   Les anticorps anti-CD 20 et anti-HLA DR chimériques produits dans YB2/0 induisent des taux plus importants d' IFN gamma en présence de leur cible (Raji) par rapport aux mêmes anticorps produits dans CHO. La quantité d'IFN gamma est bien dose dépendante de la concentration d'anticorps ajouté. A toutes les concentrations utilisées (10 à 200ng/ml) l'anticorps anti-HLA DR produit dans CHO n'induit pas de sécrétion d'IFN gamma, alors que 40ng/ml de l'anticorps produit dans YB2/0 induit environ 1000pg/ml d'IFN gamma.

Pour l'anticorps anti-CD20, il faut pour induire 300pg/ml d'IFN gamma moins de 10ng/ml de l'anticorps produit dans YB2/0 et 200ng/ml de l'anticorps produit dans CHO (figure 12).

## Revendications

1. Utilisation d'un anticorps monoclonal chimérique, humanisé ou humain optimisé **caractérisé en ce que** :
a) il est produit dans une lignée cellulaire sélectionnée pour ses propriétés de glycosylation du fragment Fc d'un anticorps, ou
b) la structure glycannique du Fcgamma a été modifiée ex vivo, et/ou
c) sa séquence primaire a été modifiée de façon à augmenter sa réactivité vis à vis des récepteurs Fc ;
ledit anticorps présentant i) un taux d'ADCC dépendant du FcγRIII (CD16) supérieur à 50 %, de préférence supérieur à 100 % pour un ratio E/T (cellules effectrices/cellules cibles) inférieur à 5/1, de préférence inférieur à 2/1, comparé au même anticorps produit dans une lignée CHO ; et ii) un taux de production d'au moins une cytokine par une cellule effectrice de type Jurkat CD16 ou issue du système immunitaire exprimant le récepteur CD16 supérieur à 50 %, 100 % ou de préférence supérieur à 200 % comparé au même anticorps produit dans une lignée CHO;
pour la préparation d'un médicament destiné au traitement de pathologies pour lesquelles le nombre de sites antigéniques, la densité antigénique sont faibles ou les antigènes sont peu accessibles aux anticorps, ou encore pour lesquelles le nombre de cellules effectrices activées ou recrutées est limité.

2. Utilisation selon la revendication 1 **caractérisée en ce que** le nombre de sites antigéniques est inférieur à 250 000, de préférence inférieur à 100 000 ou 50 000 par cellule cible.

3. Utilisation selon l'une des revendications 1 et 2, **caractérisée en ce que** lesdites cytokines libérées par les anticorps optimisés sont choisies parmi des interleukines, des interférons et des facteurs de nécrose tissulaire (TNF).

4. Utilisation selon l'une des revendications 1 et 2, **caractérisée en ce que** l'anticorps optimisé induit la sécrétion d'au moins une cytokine choisie parmi IL-1, IL-2, IL-3, IL-4, IL-5, IL-6, IL-7, IL-8, IL-9, IL-10,... TNFα, TGFβ, IP10 et IFNγ par les cellules effectrices du système immunitaire en particulier celles exprimant le récepteur CD 16.

5. Utilisation selon l'une des revendications 1 et 2, **caractérisée en ce que** l'anticorps induit la sécrétion d'IL-2 par la cellule Jurkat CD16 ou d' IFNγ et d'IL2 par les cellules effectrices du système immunitaire exprimant le récepteur CD16 pour un faible nombre de sites antigéniques présents à la surface des cellules cibles ou pour un faible nombre d'antigènes accessibles aux anticorps ou pour un faible nombre de cellules effectrices.

6. Utilisation selon l'une des revendications 1 à 5, **caractérisée en ce que** la cellule effectrice est une cellule leucocytaire, en particulier de la famille des NK (natural killer) ou une cellule du groupe monocytes-macrophages.

7. Utilisation selon l'une des revendications 1 à 5, **caractérisée en ce que** la cellule effectrice est une cellule Jurkat transfectée avec un vecteur d'expression codant pour le récepteur CD16.

8. Utilisation selon l'une des revendications 1 à 5, **caractérisée en ce que** l'anticorps optimisé est préparé après avoir été purifié et/ou modifié ex vivo par modification de la structure glycannique du fragment Fc.

9. Utilisation selon l'une des revendications 1 à 5, **caractérisée en ce que** l'anticorps optimisé est produit par des cellules de lignées de myélomes de rat, en particulier YB2/0 et ses dérivés.

10. Utilisation selon l'une des revendications 1 à 10, **caractérisée en ce que** l'anticorps optimisé présente une structure glycannique générale de type biantenné, avec des chaînes courtes, une faible sialylation, des mannoses et GlcNAc du point d'attache terminaux non intercalaires, et une faible fucosylation.

11. Utilisation selon la revendication 10, **caractérisée en ce que** l'anticorps optimisé présente un taux de GlcNac intermédiaire non nul.

12. Utilisation d'un anticorps selon l'une des revendications 1 à 11 pour la préparation d'un médicament destiné au traitement d'une pathologie choisie parmi la maladie hémolytique du nouveau-né, le Syndrome de Sézary, les leucémies myéloides chroniques, les cancers dont les cibles antigéniques sont faiblement exprimées, notamment le cancer du sein, les pathologies liées à l'environnement visant notamment les personnes exposées aux biphényles polychlorinés, les maladies infectieuses, notamment la tuberculose, le syndrome de la fatigue chronique (CFS), les infections parasitaires comme par exemple les schistosomules.
